# EUROPÄISCHE PATENTANMELDUNG

(11) **EP 4 704 387 A1**
(43) Veröffentlichungstag der Anmeldung: **04.03.2026**
(21) Anmeldenummer: 25198403.5
(22) Anmeldetag: 27.08.2025
(51) Int. Cl.: H04L 9/40, A61B 5/117, G01N 1/00, G07C 9/25

(54) **VERFAHREN ZUR VERIFIZIERUNG VON MESSERGEBNISSEN**

(30) Priorität: 28.08.2024 DE 102024124534
(71) Anmelder: Kraemer, Thilo, 64291 Darmstadt (DE)
(72) Erfinder: Kraemer, Thilo, 64291 Darmstadt (DE)
(74) Vertreter: Hamel, Armin

(57) **Zusammenfassung**

Beschrieben wird ein Verfahren zur Verifizierung von Messergebnissen von Proben, die mittels eines Messgeräts vermessen wurden. Durch dieses Verfahren wird verhindert, dass die erhaltenen Messergebnisse im Nachhinein manipuliert werden, ohne dass dies nachgewiesen werden kann.

## Beschreibung

Die Erfindung betrifft ein Verfahren zur Verifizierung von Messergebnissen nach den Merkmalen des Patentanspruchs 1.

Messgeräte dienen dazu, Proben zu vermessen. Nachdem die Proben mit einem solchen Messgerät vermessen wurden, werden die Messergebnisse einem Benutzer in der Regel in Form von Reporten bereitgestellt. Dabei ist es üblich, vor dem eigentlichen Vermessen der Proben das Messgerät zu kalibrieren und gegebenenfalls auch zu justieren.

Ein solches Messgerät kann beispielsweise ein Tablettentestgerät sein, mit dem pharmazeutische Produkte, wie beispielsweise Tabletten oder Körner, vermessen werden können. So können mit einem Tablettentestgerät Parameter wie beispielsweise die Länge, die Breite, die Masse sowie die Härte von pharmazeutischen Produkten gemessen werden. Auch kann die Qualität von Proben ermittelt werden. Nachdem die Messungen durchgeführt wurden, werden die Messergebnisse in Form eines Reports bereitgestellt.

Bisher war es üblich, den Report mit den darin enthaltenen Messergebnissen einfach in ein anderes externes Gerät, zum Beispiel einen PC, zu exportieren oder in Papierform auszudrucken. Die so erhaltenen Ergebnisse konnten aber im Nachhinein einfach manipuliert werden, zum Beispiel indem diese am PC geändert wurden.

Aufgabe der vorliegenden Erfindung ist es deshalb, ein Verfahren bereitzustellen, mit dem eine Manipulation von Messergebnissen verhindert wird.

Diese Aufgabe wird durch ein Verfahren nach den Merkmalen des Patentanspruchs 1 gelöst.

Es wird somit ein Verfahren zur Verifizierung von Messergebnissen beschrieben.

Dieses Verfahren zur Verifizierung von Messergebnissen umfasst folgende aufeinander folgende Schritte:
In einem ersten Schritt wird ein Messgerät bereitgestellt, mit dem Proben vermessen werden sollen, wobei das Messgerät eine Benutzungsverwaltungssoftware aufweist. Unter Proben wird im Folgenden eine Vielzahl von zu vermessenen Prüflingen als auch das Vermessen von nur einem Prüfling verstanden.

Bei dem Messgerät kann es sich um ein Tablettentestgerät handeln, mit dem pharmazeutische Produkte, wie zum Beispiel Tabletten oder Körner, Messungen unterworfen werden. Ein solches Tablettentestgerät weist zumindest eine Messstation-Anordnung auf, die mindestens eine Messstation umfasst. In der zumindest einen Messstation dieser Messstation-Anordnung kann eine Eigenschaft dieser pharmazeutischen Produkte (Proben) gemessen werden. Vorzugsweise weist dieses Tablettentestgerät mehrere Messstation-Anordnungen auf, so dass von diesen Proben auch mehrere Eigenschaften gemessen werden können. So kann mit einem Tablettentestgerät, das mehrere Messstation-Anordnungen aufweist, neben der Masse dieser pharmazeutischen Produkte beispielsweise noch deren Qualität, deren Breite, Länge sowie Härte vermessen werden. So kann beispielsweise eine Messstation-Anordnung, in der die Masse einer Probe gemessen werden sollen, eine Messstation in Form einer Waage aufweisen. Soll die Qualität einer Probe ermittelt werden, so kann dies in einer Messstation-Anordnung geschehen, die mehrere Messstationen aufweist, wobei jede Messstation mit einer Kamera oder mit einem NIR-Sensor ausgestattet ist.

Da Tablettentestgeräte bekannt sind, werden diese im Folgenden nicht im Detail beschrieben.

In einem zweiten Schritt muss eine erste Person (= der Benutzer), der zumindest eine Probe oder mehrere Proben (im Folgenden zur sprachlichen Vereinfachung auch als "Proben" bezeichnet) mit dem Messgerät vermessen möchte, nach Einschalten des Messgeräts, ein erstes Mal zumindest ein biometrisches Charakteristikum mittels einer dafür vorgesehenen Identifikationsvorrichtung erfassen lassen. Dieses erfasste biometrische Charakteristikum wird anschließend im Messgerät hinterlegt, indem dieses biometrische Charakteristikum in der Benutzungsverwaltungssoftware abgespeichert wird.

Als biometrisches Charakteristikum eignet sich zum Beispiel ein Fingerabdruck, die Retina oder eine Unterschrift. In den meisten Fällen dürfte es ausreichen, wenn die erste Person nur ein biometrisches Charakteristikum im Messgerät hinterlegen muss. Sollten jedoch höhere Sicherheitsanforderungen gegeben sein, so kann es erforderlich sein, mehrere biometrische Charakteristika zu hinterlegen.

Das Messgerät speichert in einem dritten Schritt dieses eine biometrische Charakteristikum oder die mehreren hinterlegten biometrischen Charakteristika in der Benutzungsverwaltungssoftware ab.

Nachdem das zumindest eine biometrische Charakteristikum abgespeichert und dadurch in dem Messgerät hinterlegt wurde, wird das Messgerät in einem vierten Schritt für die Vermessung der Proben freigeschaltet.

Spätestens dann kann eine Person Proben, die vermessen werden sollen, dem Messgerät bereitstellen. Die Proben können dann in einem fünften Schritt vermessen werden. Dabei passieren die Proben nacheinander die zumindest eine Messstation-Anordnung, wobei für jede der Proben in dieser Messstation-Anordnung ein Messwert erhalten wird. Sollte das Messgerät mehrere Messstation-Anordnungen aufweisen, so wird in jeder Messstation-Anordnung je ein Messwert für eine Probe erhalten.

In einem sechsten Schritt wird ein Messergebnis in Form von zumindest einem Messwert der vermessenen Proben in dem Messgerät als Report hinterlegt. Für jede Messstation-Anordnung wird damit ein Messergebnis erhalten, das zumindest aus einem Messwert einer Probe besteht. Werden somit mehrere Proben in der entsprechenden Messstation-Anordnung vermessen, so besteht das Messergebnis aus mehreren Messwerten, nämlich je ein Messwert für je eine Probe. Werden die Proben in einem Messgerät vermessen, das beispielsweise vier Messstation-Anordnungen besitzt, so werden für jede Probe auch vier Messergebnisse erhalten. Diese Messergebnisse werden in dem Messgerät als Report hinterlegt Der Report enthält somit mindestens ein Messergebnis.

Nachdem die Proben vermessen wurden bzw. die zumindest eine Probe vermessen wurde, muss ein zweites Mal das zumindest eine biometrische Charakteristikum erfasst und anschließend im Messgerät hinterlegt werden. Die Hinterlegung dieses zumindest einen biometrische Charakteristikum in dem Messgerät erfolgt wiederum durch Abspeichern in der Benutzungsverwaltungssoftware. Im Regelfall wird dieses biometrische Charakteristikum, das zum zweiten Mal erfasst wird, ebenfalls von der ersten Person stammen.

Dieses zum zweiten Mal erfasste und in der Benutzungsverwaltungssoftware abgespeicherte zumindest eine biometrische Charakteristikum wird durch die Benutzungsverwaltungssoftware des Messgeräts mit dem das erste Mal hinterlegten zumindest einem biometrischen Charakteristikum der ersten Person verglichen. Sind die beiden hinterlegten Charakteristika identisch, so ermöglicht das Messgerät die Weiterverarbeitung des Reports. Es ist damit klar, dass das zum ersten Mal hinterlegte biometrische Charakteristikum der ersten Person und dass das zweite Mal hinterlegte biometrische Charakteristikum identisch sein müssen, was bedeutet, dass die biometrischen Charakteristika auch von der gleichen Person stammen müssen. Sollte das zum zweiten Mal hinterlegte biometrische Charakteristikum von einer anderen Person stammen, so wird dies von der Benutzungsverwaltungssoftware erkannt. Ist das zumindest eine biometrische Charakteristikum nicht identisch, so verweigert das Messgerät die Weiterverarbeitung des Reports.

Eine Weiterverarbeitung des Reports bedeutet zum Beispiel, dass eine Person den Report für diese gerade vermessenen Proben entweder in Papierform ausdrucken kann oder zu einem externen Gerät, zum Beispiel einem PC, exportieren kann. Dadurch, dass die Messergebnisse im Messgerät abgespeichert bleiben und durch die Hinterlegung der biometrischen Charakteristika verifiziert wurden, kann eine Manipulation von Messergebnissen bzw. des gesamten Reports im Nachhinein, zum Beispiel an einem PC, jederzeit nachgewiesen werden.

Obwohl es möglich ist, die Proben bereits vorher im Messgerät bereitzustellen, so müssen die Proben spätestens, nachdem das Messgerät für die Messungen freigeschaltet wurde, bereitgestellt werden. Das Bereitstellen von Proben zu diesem Zeitpunkt ist nicht zuletzt auch deshalb sinnvoll, weil das Messgerät erst dann freigeschaltet wird und die Messungen erst dann gestartet werden können.

Damit biometrische Charakteristika überhaupt erfasst werden können, weist das Messgerät zumindest eine Identifikationsvorrichtung auf. Mit dieser Identifikationsvorrichtung kann ein bestimmtes biometrisches Charakteristikum erfasst werden. So kann es sich zum Beispiel bei der Identifikationsvorrichtung um ein Display handeln, auf dem eine Person ihre Unterschrift leisten muss.

Auch ist es möglich, als Identifikationsvorrichtung einen Scanner einzusetzen, der ein biometrisches Charakteristikum durch Vermessung erfasst. So kann mit einem solchen Scanner beispielsweise ein Fingerabdruck oder die Retina vermessen werden.

Je nachdem, wie hoch der Sicherheitsstandard sein soll, kann das Messgerät auch mehrere Identifikationsvorrichtungen aufweisen.

Nachdem das zumindest eine biometrische Charakteristikum der ersten Person erfasst und in der Benutzungsverwaltungssoftware abgespeichert wurde, kann zumindest ein biometrisches Charakteristikum einer zweiten Person ein erstes Mal erfasst werden. Dieses Charakteristikum der zweiten Person wird anschließend im Messgerät hinterlegt, indem es in der Benutzungsverwaltungssoftware abgespeichert wird. Das Hinzuziehen einer zweiten Person ist insbesondere dann vorteilhaft, wenn die Sicherheitsanforderungen sehr hoch sein sollen. Die zweite Person fungiert dann als Prüfer.

Nach Abschluss der Messungen, und nachdem das Messgerät den entsprechenden Report erstellt hat, muss das zum ersten Mal erfasste zumindest eine biometrische Charakteristikum der zweiten Person verifiziert werden. Dazu muss das zumindest eine erfasste Charakteristikum, das heißt, das gleiche Charakteristikum, ein zweites Mal erfasst werden. Dieses zum zweiten Mal erfasste zumindest eine biometrische Charakteristikum wird anschließend im Messgerät hinterlegt, indem es in der Benutzungsverwaltungssoftware abgespeichert wird. Danach vergleicht die Benutzungsverwaltungssoftware des Messgeräts das, das erste Mal hinterlegte zumindest eine biometrische Charakteristikum der zweiten Person mit dem dazu das zweite Mal hinterlegten zumindest einem biometrischen Charakteristikum. Sind die beiden Charakteristika identisch, so ermöglicht das Messgerät die Weiterverarbeitung des Reports, insofern auch die Charakteristika der ersten Person übereinstimmen. Wird hingegen durch die Benutzungsverwaltungssoftware festgestellt, dass die beiden Charakteristika nicht übereinstimmen, so ist eine Weiterverarbeitung des Reports nicht möglich, weil das Messgerät die Optionen für eine Weiterverarbeitung des Reports sperrt.

Wird somit ein Prüfer hinzugezogen, so muss nicht nur das zumindest eine biometrische Charakteristikum der zweiten Person, sondern auch das zumindest eine biometrische Charakteristikum der ersten Person identisch sein. Es erfolgt somit eine doppelte Verifizierung der Messungen, was eine Manipulation der Messergebnisse oder gar des ganzen Reports noch schwieriger gestaltet.

Für einen Fachmann ist klar, dass auch bei der Justierung oder Kalibrierung des Messgeräts und nicht nur für die eigentliche Vermessung von Proben (Chargen), Proben vermessen werden müssen. Deshalb können Reporte verschiedener Art erhalten werden, zum Beispiel ein Chargenreport, ein Kalibrierreport oder ein Justierreport, womit mit dem Verfahren auch Kalibrierreporte oder Justierreporte verifiziert und damit vor Manipulationen geschützt werden können.

## Patentansprüche

1. Verfahren zur Verifizierung von Messergebnissen, die durch Vermessen von Proben in einem Messgerät erhalten wurden, umfassend folgende aufeinander folgende Schritte
1.1 es wird ein Messgerät zur Vermessung von Proben bereitgestellt, wobei das Messgerät mindestens eine Identifikationsvorrichtung, eine Benutzungsverwaltungssoftware sowie mindestens eine Messstation-Anordnung, umfassend mindestens eine Messstation, aufweist
1.2 eine erste Person, die die Proben mit dem Messgerät vermessen möchte, muss, nach Einschalten des Messgeräts, ein erstes Mal zumindest ein biometrisches Charakteristikum von der mindestens einen Identifikationsvorrichtung erfassen lassen
1.3 das von der mindestens einen Identifikationsvorrichtung erfasste zumindest eine biometrische Charakteristikum wird in dem Messgerät hinterlegt, indem es in der Benutzungsverwaltungssoftware abgespeichert wird
1.4 das Messgerät wird für die Messung der Proben freigeschaltet
1.5 das Messgerät vermisst die Proben, wobei die Proben in der mindestens einen Messstation-Anordnung nacheinander vermessen werden, wobei für jede Probe, die in der mindestens einen Messstation-Anordnung vermessen wird, je ein Messwert erhalten wird
1.6 es wird für die mindestens eine Messstation-Anordnung ein Messergebnis in Form von zumindest einem Messwert in dem Messgerät als Report hinterlegt
1.7 es muss zu dem das erste Mal hinterlegten zumindest einem biometrischen Charakteristikum der ersten Person ein zweites Mal dieses zumindest eine biometrische Charakteristikum von der Identifikationsvorrichtung erfasst und in der Benutzungsverwaltungssoftware abgespeichert werden.

2. Verfahren nach Patentanspruch 1, **dadurch gekennzeichnet, dass** zwischen den Schritten 1.4 und 1.5 die Proben, die vermessen werden sollen, dem Messgerät bereitgestellt werden.

3. Verfahren nach Patentanspruch 1, **dadurch gekennzeichnet, dass** nach Schritt 1.7 die Benutzungsverwaltungssoftware des Messgeräts vergleicht, ob das von der ersten Person das erste Mal erfasste zumindest eine biometrische Charakteristikum mit dem das zweite Mal erfassten zumindest einem biometrischen Charakteristikum identisch ist.

4. Verfahren nach Patentanspruch 3, **dadurch gekennzeichnet, dass** das Messgerät die Weiterverarbeitung des Reports ermöglicht, wenn das, das erste Mal erfasste zumindest eine biometrische Charakteristikum der ersten Person mit dem das zweite Mal erfassten zumindest einem biometrischen Charakteristikum identisch ist, oder das Messgerät verweigert die Weiterverarbeitung des Reports, wenn das, das erste Mal erfasste zumindest eine biometrische Charakteristikum der ersten Person mit dem das zweite Mal erfassten zumindest einem biometrischen Charakteristikum nicht identisch ist.

5. Verfahren nach Patentanspruch 1, **dadurch gekennzeichnet, dass** die Identifikationsvorrichtung ein Display ist, auf dem eine Person eine Unterschrift leisten muss.

6. Verfahren nach Patentanspruch 1, **dadurch gekennzeichnet, dass** die Identifikationsvorrichtung ein Scanner ist, der das zumindest eine biometrische Charakteristikum durch Vermessung erfasst.

7. Verfahren nach Patentanspruch 1, **dadurch gekennzeichnet, dass** nach dem Schritt 1.3 ein erstes Mal zumindest ein biometrisches Charakteristikum einer zweiten Person durch die mindestens eine Identifikationsvorrichtung erfasst und anschließend durch Abspeichern in der Benutzungsverwaltungssoftware im Messgerät hinterlegt wird.

8. Verfahren nach Patentanspruch 7, **dadurch gekennzeichnet, dass** nach Schritt 1.7 zu dem das erste Mal hinterlegten zumindest einem biometrischen Charakteristikum der zweiten Person ein zweites Mal das zumindest eine biometrische Charakteristikum von der mindestens einen Identifikationsvorrichtung erfasst und in der Benutzungsverwaltungssoftware abgespeichert wird.

9. Verfahren nach Patentanspruch 8, **dadurch gekennzeichnet, dass** die Benutzungsverwaltungssoftware des Messgeräts das, das erste Mal erfasste zumindest eine biometrische Charakteristikum der zweiten Person mit dem dazu das zweite Mal erfassten zumindest einem biometrischen Charakteristikum vergleicht und das Messgerät die Weiterverarbeitung des Reports ermöglicht, wenn das, das erste Mal erfasste zumindest eine biometrische Charakteristikum der zweiten Person mit dem dazu das zweite Mal erfassten zumindest einem biometrischen Charakteristikum identisch ist, oder das Messgerät verweigert die Weiterverarbeitung des Reports, wenn das, das erste Mal erfasste zumindest eine biometrische Charakteristikum der zweiten Person mit dem dazu das zweite Mal erfassten zumindest einem biometrischen Charakteristikum nicht identisch ist und/oder wenn das, das erste Mal erfasste zumindest eine biometrische Charakteristikum der ersten Person mit dem dazu das zweite Mal erfassten zumindest einem biometrischen Charakteristikum nicht identisch ist.

10. Messgerät mit dem das Verfahren nach den Patentansprüchen 1 bis 9 durchführbar ist.

11. Messgerät nach dem Patentanspruch 10, **dadurch gekennzeichnet, dass** das Messgerät zumindest eine Messstation-Anordnung umfasst, die mindestens eine Messstation aufweist, wobei für jede Probe, die in der zumindest einen Messstation-Anordnung vermessen wird, ein Messwert erhaltbar ist.

12. Messgerät nach dem Patentanspruch 10, **dadurch gekennzeichnet, dass** das Messgerät mindestens eine Identifikationsvorrichtung, mit der ein bestimmtes biometrisches Charakteristikum erfassbar ist, aufweist.

13. Messgerät nach dem Patentanspruch 10, **dadurch gekennzeichnet, dass** das Messgerät ein Tablettentestgerät ist.
